# EUROPEAN PATENT APPLICATION

(11) **EP 4 570 902 A1**
(43) Date of publication of application: **18.06.2025**
(21) Application number: 23852521.6
(22) Date of filing: 07.08.2023
(51) Int. Cl.: C12N 5/071, A61K 35/28, A61K 35/407, A61K 35/44, A61K 35/545, A61L 27/38, A61P 1/16, A61P 43/00, C12N 5/0775

(54) **METHOD FOR PRODUCING CELL AGGREGATES**

(30) Priority: 08.08.2022 JP 2022126643
(71) Applicant: Healios K.K., Tokyo 100-0006 (JP); Public University Corporation Yokohama City University, Yokohama-shi, Kanagawa 236-0027 (JP)
(72) Inventor: INAMORI, Masakazu, Tokyo 100-0006 (JP); HINO, Motohiro, Tokyo 100-0006 (JP); AMIMOTO, Naoki, Tokyo 100-0006 (JP); TATSUMI, Rie, Tokyo 100-0006 (JP); IKEDA, Kazuhiro, Tokyo 113-0034 (JP); TANIGUCHI, Hideki, Yokohama-shi, Kanagawa 236-0004 (JP); OKAMOTO, Satoshi, Yokohama-shi, Kanagawa 236-0004 (JP)
(74) Representative: J A Kemp LLP
(86) International application number: PCT/JP2023/028720
(87) International publication number: WO 2024/034559

(57) **Abstract**

The present invention provides a method for mass producing cell aggregates such as artificial organoids through an approach that differs entirely from previous methods. Specifically, provided is a method for producing cell aggregates that includes a step in which a mixture of mesenchymal stem cells, vascular endothelial cells, and organ cells are filled into microfibers, and the mixture is floating cultured to form cell aggregates.

## Description

### FIELD

The present invention relates to a method for producing cell aggregates. In particular, the present invention relates to a method for producing cell aggregates, including the step of forming cell aggregates by filling a mixture of mesenchymal stem cells, vascular endothelial cells, and organ cells into microfibers and then subjecting the cells to floating culture within the microfibers.

### BACKGROUND

Current clinical practice employs organ transplantation or replacement with an artificial organ to treat severe organ failure. However, fundamental problems still remain unsolved, such that organ transplantation has problems of rejections and critical shortage of donors and artificial organs can replace the functions of natural organs only partially and for a short period of time. Under these circumstances, various efforts have been made to develop a method to induce artificial organoids that can withstand transplantation. For example, PTL 1 has succeeded in artificial in vitro production of organ buds, which serve as starting materials for producing tissues and organs, by artificially recapitulating early processes of organogenesis, thereby inducing early differentiation via interactions among a plurality of cell lineages and inducing the tissue-forming potential of organ cells that have undergone the early differentiation. Specifically, in PTL 1, organ buds are produced by seeding a cell suspension containing vascular endothelial cells and mesenchymal stem cells together with either hepatic endoderm cells or a pancreatic β-cell line to a culture dish coated with solidified Matrigel. However, the method disclosed in PTL 1 is not suitable for producing organ buds in large quantities.

Further, new culture methods have been developed that mimic pericellular environment or cell morphology in vivo, thereby allowing cells having functions more similar to those in vivo to be obtained. As such culture methods, methods for culturing spheroids, which are methods capable of maintaining interactions among cells, are attracting attention, and they are applied to culture of various cells such as pancreatic islet cells, hepatocytes, stem cells, and cancer cells. Examples of the methods for culturing spheroids include: a method in which cell populations are placed in round-bottom wells and allowed to stand still to form individual spheroids separately (hanging drop method; e.g., NPL 1); and a method in which spheroids are formed within droplets formed in wells provided with openings (e.g., PTL 2). However, these methods have their own advantages and also disadvantages, e.g., they are not suitable for producing spheroids in large quantities and they pose restrictions on the size of spheroids and the type of cells.

### [CITATION LIST]

### [PATENT LITERATURE]

[PTL 1] International Patent Publication No. WO2013/047639
[PTL 2] International Patent Publication No. WO2015/129263

### [NON PATENT LITERATURE]

[NPL 1] Sasitorn Rungarunlert et al., "Embryoid body formation from embryonic and induced pluripotent stem cells: Benefits of bioreactors", World Journal of Stem Cells 1 (1), December 31, 2009, pp. 11-21

### SUMMARY

### [TECHNICAL PROBLEM]

With the foregoing in mind, it is an object of the present invention to provide a method for producing cell aggregates such as artificial organoids in large quantities through an approach that is totally different from conventional methods.

### [SOLUTION TO PROBLEM]

The inventors of the present invention made an attempt to develop a method capable of efficiently producing cell aggregates, such as organoids, containing a plurality of types of cells by improving floating culture conventionally used to produce spheroids. However, it had been known that conventional methods for culturing spheroids cannot produce desired cell aggregates containing a plurality of types of cells because aggregation of the same types of cells occurs when a cell population containing a plurality of types of cells is subjected to suspension culture (e.g., Junji Fukuda, Research Report 2019 (KISTEC Annual Research Report, 2019, Takechi et al, DEVELOPMENTAL BIOLOGY 37,340-350 (1981)). In light of the foregoing, the inventors conducted intensive studies and came up with the idea that, by filling a mixture of a plurality of types of cells into microfibers and subjecting the cells to floating culture within the microfibers, it might be possible to prevent aggregation of the same type of cells, thereby allowing cell aggregates containing a plurality of types of cells to be obtained. Based on this idea, the inventors filled a mixture of mesenchymal stem cells, vascular endothelial cells, and organ cells into microfibers and subjected the cells to floating culture. As a result, the inventors succeeded in obtaining cell aggregates in which the respective types of cells were evenly distributed, and still more surprisingly, the thus-obtained cell aggregates were found to be structures that can be considered as organoids having a new function that is not found in the original cells (e.g., albumin secretion ability). The inventors conducted further studies based on this finding, which led to completion of the present invention.

Specifically, the present invention provides the following.
[1] A method for producing a cell aggregate, including the step of:
   forming a cell aggregate by filling a mixture of mesenchymal stem cells, vascular endothelial cells, and organ cells into a microfiber and subjecting the cells to floating culture within the microfiber.
[2] The method according to claim [1], wherein the cell aggregate is an organoid.
[3] The method according to [1] or [2], wherein the organ cells are endodermal cells.
[4] The method according to any one of [1] to [3], wherein the endodermal cells are hepatocytes.
[5] The method according to any one of [1] to [4], wherein the microfiber has an inner diameter from 20 to 300 µm.
[6] The method according to any one of [1] to [5], wherein a cell density in the microfiber filled with the cells is from 5 × 10⁶ cells/mL to 4 × 10⁸ cells/mL.
[7] The method according to any one of [1] to [6], wherein a proportion of the mesenchymal stem cells in a cell population filling the microfiber is not less than 1% and not more than 20%.
[8] The method according to any one of [1] to [7], wherein a proportion of the vascular endothelial cells in a cell population filling the microfiber is not less than 10% and not more than 60%.
[9] The method according to any one of [1] to [8], wherein at least one type of cells selected from the mesenchymal stem cells, the vascular endothelial cells, and the organ cells are derived from induced pluripotent stem cells.
[10] A cell aggregate produced by the method according to any one of [1] to [9].
[11] An agent for transplantation therapy, containing:
   the cell aggregate according to [10].
[12] An agent for transplantation therapy according to [11] for treating damage to a tissue or an organ or a disease.
[13] A method for treating damage to a tissue or an organ or a disease, including:
   administering or transplanting an effective amount of the cell aggregate according to [10] to a subject.
[14] The cell aggregate according to [10] for use in treatment of damage to a tissue or an organ or a disease.
[15] Use of the cell aggregate according to [10] in production of a medicine for treating damage to a tissue or a disease.

### [ADVANTAGEOUS EFFECTS OF INVENTION]

According to the present invention, cell aggregates containing a plurality of types of cells can be produced in large quantities. Besides, the cell aggregates produced by the present invention are capable of functioning as organoids and thus can be useful as an agent for transplantation therapy for use in vivo.

### BRIEF DESCRIPTION OF DRAWINGS

FIG. 1 shows the results of fluorescence microscope observation of spheroids produced using an Elplasia plate.
FIG. 2 shows the results of fluorescence microscope observation of spheroids produced using hollow microfibers.
FIG. 3 compares the amounts of albumin secretion per unit cells in respective spheroids.

### DESCRIPTION OF EMBODIMENTS

### 1. Method for producing cell aggregates

The present invention relates to a method for producing cell aggregates, including the step of culturing a plurality of types of cells under floating culture conditions. More specifically, the present invention relates to a method for producing cell aggregates, including the steps of: forming cell aggregates by filling a mixture of mesenchymal stem cells, vascular endothelial cells, and organ cells into microfibers and then subjecting the cells to floating culture within the microfibers (hereinafter, the method may also be referred to as "the production method of the present invention"). In another aspect, the present invention provides a cell aggregate obtained by the production method of the present invention.

As used herein, the term "microfiber" refers to a structure having a core portion and a sheath-like shell (covering) portion that covers the core portion, and such a microfiber is typically a hollow microfiber. In the production method of the present invention, mesenchymal stem cells, vascular endothelial cells, and organ cells are subjected to floating culture in a hollow portion (i.e., core portion) of such a microfiber. Accordingly, the core portion contains not only the respective types of cells but also a medium and the like to be described below. The sheath-like shell portion may have a single-layer structure containing a material to be described below, or may have a multi-layer structure with two or more layers.

The material used for the sheath-like shell portion is not limited to a particular material as long as the sheath-like shell portion can have a strength sufficient to hold the core portion, and may be, for example, alginate or agarose. More specifically, the material may be, for example, sodium alginate, potassium alginate, ammonium alginate, or any combination thereof. In addition, the material may also be, for example, a mixture of an alginate with agar, agarose, polyethylene glycol, polylactic acid, nanocellulose, or the like. Alginic acid may be a natural extract or a chemically modified alginic acid. Examples of the chemically modified alginic acid include methacrylate-modified alginic acid.

The material used for the sheath-like shell portion may be a material that forms a gel upon contact with the above-described alginates, agarose, or the like. Specific examples of such a material include polyvalent cationic materials, and more specific examples thereof include calcium chloride and barium chloride.

The inner diameter of the microfibers to be used in the production method of the present invention, i.e., the inner diameter of the shell portion, can be set as appropriate based on the size of cell aggregates intended to be obtained etc., and is, for example, approximately from 10 µm to 500 µm and preferably approximately from 20 µm to 300 µm. The cross-sectional shape of the microfibers is desirably circular, but also may be oval or polygonal, e.g., quadrilateral or pentagonal. The length of the microfibers can be set as appropriate based on the size of cell aggregates intended to be obtained etc., and is, for example, approximately from 1 mm to 100 m. The outer diameter of the microfibers, i.e., the outer diameter of the shell portion, can be set as appropriate based on the size of cell aggregates intended to be obtained etc., and is, for example, approximately from 10 µm to 2000 µm.

The material used for the shell portion of such microfibers is desirably sodium alginate and calcium chloride, and the shell portion desirably has a circular cross-sectional shape with an inner diameter approximately from 50 to 200 µm.

Such microfibers can be produced by known methods (e.g., Japanese Unexamined Patent Publication Nos. 2017-99303 and 2021-016319). Although not wishing to be bound by any theory, the reason why culturing cells in microfibers allows cell aggregates containing a plurality of types of cells to be obtained while preventing the aggregation of the same type of cells is speculated as follows: in the microfibers, neighboring cells contained in the mixture of cells can stay together without dispersing, whereby even different types of cells exhibiting different adhesion strengths can gather to form aggregates. Therefore, microfibers other than those used in examples of the present invention are also applicable to the present invention.

As used herein, the term "floating culture conditions" refers to conditions under which cells or aggregates of cells remain in a state where they are floating in a liquid medium, i.e., conditions that do not allow direct or indirect strong cell-substratum junctions to be formed between cells or aggregates of cells and the inner wall of the core portion of the microfibers.

In the production method of the present invention, the proportion of the organ cells in a cell population filling the microfibers can be set as appropriate based on the size and properties of cell aggregates intended to be obtained etc., and is, for example, not less than 30% and not more than 90% and preferably not less than 45% and not more than 77%.

The medium to be used under the floating culture conditions can be prepared by adding a medium additive(s) to a basal medium as needed. Examples of the basal medium include, but are not limited to: a RPMI-1640 medium, Eagle's MEM (EMEM), Dulbecco's modified MEM (DMEM), Glasgow's MEM (GMEM), α-MEM, 199 medium, IMDM, Hybridoma Serum free medium, KnockOut^{™} DMEM (KO DMEM), Advanced^{™} media (e.g., Advanced MEM, Advanced RPMI, and Advanced DMEM/F-12), Chemically Defined Hybridoma Serum Free medium, Ham's Medium F-12, Ham's Medium F-10, Ham's Medium F12K, DMEM/F-12, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's Medium (e.g., Waymouth's MB752/1), CMRL medium (e.g., CMRL-1066), Williams' medium E, Brinster's BMOC-3 Medium, E8 Medium, StemPro 34, and MesenPRO RS (all from Thermo Fisher Scientific); ReproFF2, Primate ES Cell Medium, and ReproStem (all from ReproCELL Incorporated); Procul AD (ROHTO Pharmaceutical Co., Ltd.); MSCBM-CD and MSCGM-CD (both from Lonza); EX-CELL 302 medium (SAFC) or EX-CELL-CD-CHO (SAFC); ReproMed^{™} iPSC Medium (ReproCELL Incorporated); Cellartis MSC Xeno-Free Culture Medium (Takara Bio Inc.); TESR-E8 (Veritas Corporation); StemFit^{®} AK02N and AK03N (Ajinomoto Co., Inc.); and mixtures thereof.

The medium may be supplemented with biologically active substances, nutritional factors, etc. required for survival or growth of cells, as needed. Such medium additives may be added to the medium beforehand or may be added during cell culture. The method of adding such additives during culture may be in any form, e.g., adding the additives in the form of solutions each containing one type of additive or in the form of a mixed solution containing two or more additives, and they may be added continuously or intermittently.

Examples of the biologically active substances include insulin, IGF-1, transferrin, albumin, coenzyme Q10, various types of cytokines (such as interleukins (IL-2, IL-7, IL-15, etc.), stem cell factor (SCF), and activin), various types of hormones, and various types of growth factors (such as leukemia inhibitory factor (LIF), basic fibroblast growth factor (bFGF), and TGF-β). Examples of the nutritional factors include sugars, amino acids, vitamins, hydrolysates, and lipids. Examples of the sugars include glucose, mannose, and fructose. One type of sugar may be used, or two or more types of sugars may be used in combination. Examples of the amino acids include L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine. One type of amino acid may be used, or two or more types of amino acids may be used in combination. Examples of the vitamins include d-biotin, D-pantothenic acid, choline, folic acid, myo-inositol, niacinamide, pyridoxal, riboflavin, thiamine, cyanocobalamin, and DL-α-tocopherol. One type of vitamin may be used, or two or more types of vitamins may be used in combination. Examples of the hydrolysates include those obtained by hydrolyzing soybeans, wheat, rice, peas, corn, cottonseeds, and yeast extracts. Examples of the lipids include cholesterol, linoleic acid, and linolenic acid. Examples of polysaccharides include gellan gum, deacylated gellan gum, methylcellulose, carboxymethylcellulose, hydroxyethylcellulose, hydroxypropylcellulose, carboxymethylamylose, xanthan gum, alginic acid, carrageenan, diutan gum, and locust bean gum.

The medium may be further supplemented with an antibiotic such as kanamycin, streptomycin, penicillin, or hygromycin, as needed. When an acidic substance such as sialic acid is added to the medium, it is desirable to adjust the pH of the medium to a value in a neutral range suitable for cell growth, namely, pH 5 to 9 and preferably pH 6 to 8.

The medium to be used for floating culture may be a medium containing a serum (e.g., fetal bovine serum (FBS), human serum, or horse serum) or a serum-free medium. The serum is preferably FBS. From the viewpoint of preventing contamination with components derived from different animal species, it is preferable that the medium does not contain a serum or that a serum to be used is derived from the same animal species as the source animal species of the cells to be cultured. The serum-free medium as used herein refers to a medium that does not contain unadjusted or unpurified serum. The serum-free medium may contain purified blood-derived components or animal tissue-derived components (e.g., growth factors).

The medium used for floating culture may or may not contain a serum replacement, as with serum. Examples of the serum replacement include albumin, albumin replacements such as lipid-rich albumin and recombinant albumin, plant starch, dextran, protein hydrolysates, transferrin as well as other iron transporters, fatty acid, insulin, collagen precursors, trace elements, 2-mercaptoethanol, 3'-thioglycerol, and equivalents thereof. Specific examples of the serum replacement include those prepared according to the method described in International Patent Publication No. WO98/30679 and commercially available serum replacements such as Knockout Serum Replacement [KSR] (Life Technologies), Chemically-defined Lipid concentrated (Life Technologies), and L-alanine-L-glutamine dipeptide (e.g., Glutamax (Life Technologies)). Examples of factors of biological origin include platelet-rich plasma (PRP) and components contained in a culture supernatant of human mesenchymal stem cells.

From the viewpoint of efficient production of cell aggregates, the medium used for floating culture preferably contains any of the above-described polysaccharides, in particular, methylcellulose. The concentration of the methylcellulose in the medium is not limited to a particular concentration, and may be, for example, from 0.05% to 3% (w/v) and preferably 0.3% (w/v).

As used herein, the term "organ cells" refers to functional cells that constitute an organ or undifferentiated cells that differentiate into functional cells. "Undifferentiated organ cells" may be, for example, cells capable of differentiating into an organ such as the kidney, heart, lungs, spleen, liver, esophagus, stomach, thyroid gland, parathyroid gland, thymus gland, gonads, brain, and spinal cord, and examples of such cells include: cells capable of differentiating into ectodermal organs such as the brain, spinal cord, adrenal medulla, epidermis, hair, nail, cutaneous gland, sense organs, peripheral nerves, and crystalline lens; cells capable of differentiating into mesodermal organs such as the spleen, kidney, ureter, heart, blood, gonads, adrenal cortex, muscles, skeleton, dermis, connective tissues, and mesothelium; and cells capable of differentiating into endodermal organs such as the liver, pancreas, digestive tract (pharynx, esophagus, stomach, and intestinal tract), lungs, thyroid gland, parathyroid gland, urinary tract, and thymus gland. Whether a certain type of cells are capable of differentiating into an ectodermal organ, mesodermal organ, or endodermal organ can be determined by examining the expression of proteins that serve as markers therefor (if any one or more of marker proteins are expressed, the cells can be determined as capable of differentiating into an endodermal organ). For example, markers for cells capable of differentiating into the liver include HHEX, SOX2, HNF4A, AFP, and ALB; markers for cells capable of differentiating into the pancreas include PDX1, SOX17, and SOX9; markers for cells capable of differentiating into the intestinal tract include CDX2 and SOX9; markers for cells capable of differentiating into the kidney include SIX2 and SALL1; markers for cells capable of differentiating into the heart include NKX2-5 MYH6, ACTN2, MYL7, and HPPA; markers for cells capable of differentiating into blood include C-KIT, SCA1, TER119, and HOXB4; and markers for cells capable of differentiating into the brain and spinal cord include HNK1, AP2, and NESTIN. Of the terms used by those skilled in the art, terms such as hepatoblast, hepatic progenitor cells, pancreatoblast, hepatic precursor cells, pancreatoblast, pancreatic progenitors, pancreatic progenitor cells, pancreatic precursor cells, endocrine precursors, intestinal progenitor cells, intestinal precursor cells, intermediate mesoderm, metanephric mesenchymal precursor cells, multipotent nephron progenitor, renal progenitor cell, cardiac mesoderm, cardiovascular progenitor cells, and cardiac progenitor cells (JR. Spence, et al. Nature.; 470 (7332): 105-9. (2011), Self, et al. EMBO J.; 25(21): 5214-5228. (2006), J. Zhang, et al. Circulation Research.; 104: e30-e41 (2009), and G. Lee, et al. Nature Biotechnology 25, 1468-1475 (2007)) are encompassed in the undifferentiated organ cells in the present invention. Undifferentiated organ cells can be produced from pluripotent stem cells such as induced pluripotent stem cells (iPS cells) and embryonic stem cells (ES cells) according to known methods. For example, organ cells capable of differentiating into the liver can be produced according to K. Si-Taiyeb, et al. Hepatology, 51 (1): 297- 305 (2010) or T. Touboul, et al. Hepatology. 51(5): 1754-65. (2010), organ cells capable of differentiating into the pancreas can be produced according to D. Zhang, et al. Cell Res.; 19(4): 429-38. (2009), and organ cells capable of differentiating into the intestinal tract can be produced according to J. Cai, et al. J Mol Cell Biol.; 2(1): 50-60 (2010) or R. Spence, et al. Nature.; 470 (7332): 105-9. (2011), organ cells capable of differentiating into the heart can be produced according to J. Zhang, et al. Circulation Research.; 104: e30-e41 (2009), and organ cells capable of differentiating into the brain and spinal cord can be produced according to G. Lee, et al. Nature Biotechnology 25, 1468-1475 (2007). Examples of "differentiated organ cells" include endocrine cells in the pancreas, pancreatic duct epithelial cells in the pancreas, hepatocytes in the liver, epithelial cells in the intestinal tract, tubular epithelial cells in the kidney, glomerular epithelial cells in the kidney, myocardial cells in the heart, lymphocytes, granulocytes, and erythrocytes in blood, neurons and glia cells in the brain, and neurons and Schwann cells in the spinal cord. As the organ cells, those derived from humans are mainly used. Also, animal cells from animals other than humans, e.g., rodents such as rats, mice, hamsters, guinea pigs, lagomorphs such as rabbits, ungulates such as pigs, cows, goats, and sheep, dogs, felines such as cats, and primates such as monkeys, rhesus monkeys, marmosets, orangutans, and chimpanzees, may be used as the organ cells.

In the production method of the present invention, the organ cells may be produced (induced to differentiate) from pluripotent stem cells. For example, liver progenitor cells (HE) can be induced by culturing pluripotent stem cells in a medium containing Activin A to induce them into an endoderm and then culturing the endoderm in a medium containing BMP (such as BMP-4) and FGF (such as bFGF or FGF4).

As used herein, the term "vascular endothelial cells" refers to cells that constitute a vascular endothelium or cells capable of differentiating into such cells. Whether a certain type of cells are vascular endothelial cell can be determined by examining whether marker proteins such as, for example, TIE2, VEGFR-1, VEGFR-2, VEGFR-3, and CD31 are expressed (if any one or more of these marker proteins are expressed, the cells can be determined as vascular endothelial cells). The vascular endothelial cells used in the present invention may be either in a differentiated state or an undifferentiated state. Whether the vascular endothelial cells are differentiated cells can be determined by examining CD31 and CD144. Of the terms used by those skilled in the art, terms such as endothelial cells, umbilical vein endothelial cells, endothelial progenitor cells, endothelial precursor cells, vasculogenic progenitors, and hemangioblasts (HJ. joo, et al. Blood. 25; 118(8): 2094-104. (2011)) are encompassed in the vascular endothelial cells of the present invention. As the vascular endothelial cells, those derived from humans are mainly used. Also, the vascular endothelial cells may be those derived from animals other than humans, e.g., rodents such as rats, mice, hamsters, guinea pigs, lagomorphs such as rabbits, ungulates such as pigs, cows, goats, and sheep, dogs, felines such as cats, and primates such as monkeys, rhesus monkeys, marmosets, orangutans, and chimpanzees.

In the production method of the present invention, the vascular endothelial cells may be produced (induced to differentiate) from pluripotent stem cells. For example, the vascular endothelial cells can be induced by culturing pluripotent stem cells in a medium containing BMP (such as BMP-4) to induce them into a mesoderm and then culturing the mesoderm in a medium containing VEGF.

As used herein, the term "mesenchymal stem cells" refers to stem cells capable of differentiating into cells that belong to the mesenchymal lineage. Whether a certain type of cells are mesenchymal stem cells can be determined by examining whether marker proteins such as, for example, Stro-1, CD29, CD44, CD73, CD90, CD105, CD133, CD271, and Nestin are expressed (if any one or more of these marker proteins are expressed, the cells can be determined as mesenchymal stem cells). Of the terms used by those skilled in the art, terms such as mesenchymal stem cells and mesenchymal progenitor cells (R. Peters, et al. PLoS One. 30; 5(12): e15689. (2010)) are encompassed in the mesenchymal stem cells of the present invention. As the mesenchymal stem cells, those derived from humans are mainly used. Also, the mesenchymal stem cells may be those derived from animals other than humans, e.g., rodents such as rats, mice, hamsters, guinea pigs, lagomorphs such as rabbits, ungulates such as pigs, cows, goats, and sheep, dogs, felines such as cats, and primates such as monkeys, rhesus monkeys, marmosets, orangutans, and chimpanzees.

In the production method of the present invention, the mesenchymal stem cells may be produced (induced to differentiate) from pluripotent stem cells. For example, the mesenchymal stem cells can be induced by culturing pluripotent stem cells in a medium containing BMP (such as BMP-4), then in a medium containing PDGF-BB and Activin ABMP, and thereafter in a medium containing BMP (such as BMP-4).

The term "pluripotent stem cells" refers to stem cells that can differentiate into tissue or cells having various different forms or functions in the body, and that can also differentiate into cells of any lineages of the three germ layers (endoderm, mesoderm and ectoderm). Examples of pluripotent stem cells to be used for the present invention include induced pluripotent stem cells (iPS cells), embryonic stem cells (ES cells), embryonic stem cells from cloned embryos obtained by nuclear transfer (nuclear transfer Embryonic stem cells; ntES cells), multipotent germline stem cells (mGS cells) and embryonic germ cells (EG cells), of which iPS cells (and especially human iPS cells) are preferred. When the pluripotent stem cells are ES cells or arbitrary cells derived from human embryos, the cells may be obtained by destruction of the embryo or they may be obtained without destruction of the embryo, but preferably they are cells obtained without destruction of the embryo.

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

ntES cells are ES cells derived from a clone embryo prepared by nuclear transfer, and they have approximately the same properties as fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). In other words, ntES (nuclear transfer ES) cells are ES cells established from the inner cell mass of a cloned embryo-derived blastocyst obtained by exchanging an unfertilized egg nucleus with a somatic cell nucleus. Combinations of nuclear transfer (Cibelli J.B. et al. (1998), Nature Biotechnol., 16:642-646) and ES cell preparation techniques (described above) are used to prepare ntES cells (Wakayama, S. (2008), Jikken Igaku, Vol. 26, No. 5 (special edition), pp. 47-52). For nuclear transfer, a somatic cell nucleus may be implanted into a mammalian enucleated unfertilized egg and cultured for several hours for reprogramming.

The ES cell line used for the present invention may be mouse ES cells, and for example, the different mouse ES cell lines established by the inGenious Targeting Laboratory, Riken (Riken Research Institute), etc., may be used, or for human ES cell lines, the different human ES cell lines established by the University of Wisconsin, NIH, Riken, Kyoto University, the National Center for Child Health and Development, Cellartis, etc., for example, may be used. Specific examples of human ES cell lines include CHB-1 to CHB-12 lines, RUES1 line, RUES2 line and HUES1 to HUES28 lines, etc., distributed by ESI Bio Co., H1 and H9 lines, etc., distributed by WiCell Research, KhES-1 line, KhES-2 line, KhES-3 line, KhES-4 line, KhES-5 line, SSES1 line, SSES2 line and SSES3 line distributed by Riken, etc.

iPS cells are cells obtained by reprogramming of mammalian somatic cells or undifferentiated stem cells by transfer of specific factors (nuclear reprogramming factors). A large number of iPS cells currently exist, among which there may be used iPSCs established by transfer of the 4 factors Oct3/4·Sox2·Klf4·c-Myc into mouse fibroblasts by Yamanaka et al. (Takahashi K, Yamanaka S., Cell, (2006) 126: 663-676), iPSCs derived from human cells established by transfer of the same 4 factors into human fibroblasts (Takahashi K, Yamanaka S., et al. Cell, (2007) 131:861-872.), Nanog-iPSCs established by selecting of Nanog expression markers after transfer of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), and iPSCs established by transfer of 6 factors by a virus-free method (Okita K et al. Nat. Methods 2011 May; 8(5):409-12, Okita K et al. Stem Cells. 31(3):458-66.), and so on. The induced pluripotent stem cells established by transfer of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), and so on, may also be used.

In addition, any induced pluripotent stem cells publicly known in the field as described in published journal (for example, Shi Y., Ding S., et al., Cell Stem Cell, (2008) Vol. 3, Issue 5, 568-574;, Kim JB., Scholer HR., et al., Nature, (2008) 454, 646-650; Huangfu D., Melton, DA., et al., Nature Biotechnology, (2008) 26, No 7, 795-797), or patents (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used.

Induced pluripotent stem cell lines that are iPSC lines established by NIH, Riken, Kyoto University, Lonza, etc., for example, may be used. Examples include human iPSC lines such as HiPS-RIKEN-1A line, HiPS-RIKEN-2A line, HiPS-RIKEN-12A line and Nips-B2 line, etc., by Riken, and 253G1 line, 253G4 line, 1201C1 line, 1205D1 line, 1210B2 line, 1383D2 line, 1383D6 line, 201B7 line, 409B2 line, 454E2 line, 606A1 line, 610B1 line, 648A1 line, 1231A3 line, FfI-01s04 line and QHJI01s04 line by Kyoto University, TC-1133HKK_05G line, TC-1133HKK_06E line by Lonza, or iPSC lines obtained by genetically modifying the above-mentioned iPSC strains, etc.

The source species of the pluripotent stem cells is not particularly limited, and for example, the cells may be from a rodent such as a rat, mouse, hamster or guinea pig, a lagomorph such as a rabbit, an ungulate such as a pig, cow, goat or sheep, a feline such as a dog or cat, or a primate such as a human, monkey, rhesus monkey, marmoset, orangutan or chimpanzee. The preferred source species is human.

The term "cells" as used herein includes "cell populations", unless otherwise specified. A cell population may be composed of a single type of cell or two or more different types of cells.

The term "cell aggregate" as used herein refers to a structure containing the following three types of cells: organ cells or cells derived therefrom; vascular endothelial cells or cells derived therefrom; and mesenchymal stem cells or cells derived therefrom. Such a structure includes portions where different cells are adjacent to each other inside. According to the production method of the present invention, cell aggregates can be formed by filling a mixture of mesenchymal stem cells, vascular endothelial cells, and organ cells into the above-described microfibers and allowing them to stand still.

In the production method of the present invention, the cell density in the microfibers filled with the cells can be set as appropriate based on the size of cell aggregates intended to be obtained etc., and is, for example, 5 × 10⁶ cells/mL to 4 × 10⁸ cells/mL and preferably 1 × 10⁷ cells/mL to 1 × 10⁸ cells/mL.

In the production method of the present invention, the proportion of the mesenchymal stem cells in a cell population filling the microfibers can be set as appropriate based on the size and properties of cell aggregates intended to be obtained etc., and is, for example, not less than 1% and not more than 20% and preferably not less than 4% and not more than 15%.

In the production method of the present invention, the proportion of the vascular endothelial cells in a cell population filling the microfibers can be set as appropriate based on the size and properties of cell aggregates intended to be obtained etc., and is, for example, not less than 10% and not more than 60% and preferably not less than 14% and not more than 48%.

In the production method of the present invention, the proportion of the organ cells in a cell population filling the microfibers can be set as appropriate based on the size and properties of cell aggregates intended to be obtained etc., and is, for example, not less than 30% and not more than 90% and preferably not less than 45% and not more than 77%.

As used herein, the term "organoid" (also referred to as "organ bud") refers to a cell aggregate that is a structure having a new function that is not found in the starting cells themselves used in the production method of the present invention. Preferably, the structure is capable of differentiating into an organ through maturation, and whether the structure has such differentiation ability can be determined by, for example, transplanting the structure into a living body and examining whether it can differentiate into an intended organ (if it differentiates into the intended organ, it can be determined that the structure is an organoid).

The organoid may be, for example, an organoid that differentiates into an organ such as the kidney, liver, heart, lungs, spleen, esophagus, stomach, thyroid gland, parathyroid gland, thymus, gonads, brain, or spinal cord. Specifically, the organoid may be, for example, a lung organoid, liver organoid, airway epithelial organoid, intestinal organoid, pancreatic organoid, kidney organoid, brain organoid, airway organoid, stomach organoid, thyroid organoid, thymus organoid, testicular organoid, esophageal organoid, skin organoid, nerve organoid, oviduct organoid, ovarian organoid, salivary gland organoid, optic vesicle organoid, optic cup organoid, bladder organoid, prostate organoid, cartilage organoid, heart organoid, bone tissue organoid, muscle tissue organoid and cancer organoid. Whether a certain structure is an organoid that differentiates into an organ can be determined by examining the expression of marker proteins. For example, markers for a liver organoid include HHEX, SOX2, HNF4A, AFP, and ALB; markers for a pancreatic organoid include PDX1, SOX17, and SOX9; markers for an organoid that differentiates into the intestinal tract include CDX2 and SOX9; and markers for a kidney organoid include Pax2 and Six2. Of the terms used by those skilled in the art, terms such as liver bud, liver diverticula, liver organoid, pancreatic (dorsal or ventral) buds, pancreatic diverticula, pancreatic organoid, intestinal bud, intestinal diverticula, and intestinal organoid (K. Matsumoto, et al. Science. 19; 294(5542): 559-63. (2001)) are encompassed in the organoid in the present invention.

Regarding the production of the above-described organoids, for example, liver organoids can be produced based on known methods (e.g., WO2013/047639 and WO2019/087988). Specifically, liver organoids can be produced by, for example, inducing liver progenitor cells (organ cells), mesenchymal stem cells, and vascular endothelial cells from pluripotent stem cells, filling a mixture of these cells into microfibers, and subjecting the cells to floating culture.

For example, pancreatic organoids can be produced based on known methods (e.g., WO2013/047639, WO2015/178431, and WO2017/047797). Specifically, pancreatic organoids can be produced by, for example, inducing pancreatic β-cells (organ cells), mesenchymal stem cells, and vascular endothelial cells from pluripotent stem cells, filling a mixture of these cells in microfibers, and subjecting the cells to floating culture.

For example, kidney organoids can be produced with reference to known methods (e.g., WO2019/230737, WO2020/022261, Taguchi & Nishinakamura, 2017, Cell Stem Cell 21, 730-746 December 7, 2017, and Tsujimoto et al., 2020, Cell Reports 31, 107476 April 7, 2020). Specifically, kidney organoids can be produced by, for example, inducing collecting duct progenitor cells (organ cells), nephron progenitor cells (organ cells), mesenchymal stem cells, and vascular endothelial cells from pluripotent stem cells, filling a mixture of these cells in microfibers, and subjecting the cells to floating culture.

Organoids having a more complex structure can be included using one of the above-described organoids alone or using two or more of the above-described organoids in combination. In the production method of the present invention, the cell aggregates formed in the microfibers can be collected using a method such as dissolving the microfibers with a chelating agent such as EDTA.

In the production method of the present invention, the temperature during the culture is not limited to a particular temperature as long as desired cell aggregates can be obtained, and may be, for example, from 30°C to 40°C and is preferably 37°C.

In the production method of the present invention, the culture period is not limited to a particular time period as long as desired cell aggregates can be obtained, and may be, for example, 1 to 14 days and is preferably 3 to 7 days.

An organoid produced by the production method of the present invention may be transplanted into a non-human animal and allowed to mature in the non-human animal to produce a tissue or an organ. Examples of the non-human animal that can be used include mice, rabbits, pigs, dogs, and monkeys. The non-human animal used for maturing the organoid is preferably immunodeficient to avoid immunorejection.

### 2. Agent for transplantation therapy

The cell aggregates obtained by the production method of the present invention (hereinafter also referred to as "the cell aggregates of the present invention") are typically structures that can be said to be organoids having a new function (e.g., albumin secretion ability) that is not found in the starting cells used in the production method of the present invention and can be tissues or organs by maturing the structures in vivo. Accordingly, the cell aggregates of the present invention can be suitably used in cell transplantation therapy. Therefore, in still another aspect, the present invention provides an agent for cell transplantation therapy, containing the cell aggregates of the present invention (hereinafter also referred to as "the agent for cell transplantation therapy according to the present invention"). The present invention also encompasses a method for treating damage to a tissue or an organ (including defects in a tissue or an organ) or a disease, including administering or transplanting an effective amount of the cell aggregates of the present invention into a mammal (e.g., a human, mouse, rat, monkey, cow, horse, pig, or dog) as a subject for the treatment. Further, "treating damage to a tissue or an organ" encompasses regenerating a damaged tissue or organ.

When the cell aggregates are transplanted into a tissue or an organ, the transplantation site may be any site as long as the transplantation is possible. The transplantation site may be, for example, in an intracranial space, in the mesentery, in the liver, in the spleen, in the kidney, under the renal capsule, or on the portal vein. In the case of performing transplantation, cell aggregates produced by the production method of the present invention may be transplanted, or alternatively, a tissue or an organ may be produced by maturing a cell aggregate produced by the production method of the present invention in a non-human animal and the thus-obtained tissue or organ may be transplanted.

When the cell aggregates of the present invention are used for cell transplantation therapy, starting cells to be used in the production method of the present invention are desirably cells derived from iPS cells established from somatic cells having the same or substantially the same HLA genotype as the recipient individual or genetically modified iPS cells from the viewpoint of preventing rejection. The term "substantially the same" in this context means that the HLA genotype identity of the somatic cells to the transplanted cells is high enough to allow an immune response to be suppressed with the use of an immunosuppressive agent, and the somatic cells have, for example, an HLA genotype that matches the HLA genotype of the transplanted cells in three loci, namely, HLA-A, HLA-B, and HLA-DR, or in four loci, namely, HLA-C in addition to these three loci. If it is difficult to obtain satisfactory cells owing to the age or body constitution, cell aggregates collected from microfibers may be transplanted in the state of being encapsulated in a capsule made of a material such as polyethylene glycol or silicone, a porous container, or the like to avoid rejection.

If necessary, the cell aggregates of the present invention may be produced in the form of a parenteral formulation such as an injection, suspension, or infusion by mixing with a pharmaceutically acceptable carrier according to a routine procedure. Therefore, in still another aspect, the present invention also provides a method for producing an agent for cell transplantation therapy, including formulating the cell aggregates of the present invention. Such a production method may include the step of preparing the cell aggregates of the present invention. Such a production method may further include the step of storing the cell aggregates of the present invention.

Examples of the pharmaceutically acceptable carrier that may be contained in the parenteral formulation include aqueous solutions for injection, such as physiological saline and isotonic solutions containing dextrose and other auxiliary agents (e.g., D-sorbitol, D-mannitol, and sodium chloride). The agent for cell transplantation therapy according to the present invention may be blended with, for example, a buffer (e.g., phosphate buffer or sodium acetate buffer), a soothing agent (e.g., benzalkonium chloride or procaine hydrochloride), a stabilizer (e.g., human serum albumin or polyethylene glycol), a preservative, and/or an antioxidant. The amount of the agent for cell transplantation therapy according to the present invention to be administered or transplanted and the frequency of administration or transplantation of the same can be determined as appropriate according to the age, weight, symptoms, etc. of a mammal to which the agent is administered.

The agent for cell transplantation therapy according to the present invention may be supplied in a state of being cryopreserved under conditions normally used for cryopreservation of cells or cell aggregates and may be thawed before use. In this case, the agent may further contain serum or its replacement, an organic solvent (e.g., DMSO), and the like. In this case, the concentration of serum or its replacement is not limited to a particular concentration, and may be from about 1% to about 30% (v/v) and preferably from about 5% to about 20% (v/v). The concentration of the organic solvent is not limited to a particular concentration, and may be from 0% to about 50% (v/v) and preferably from about 5% to about 20% (v/v).

The present invention will be described more specifically with reference to the following examples. It is to be noted, however, that the present invention is not limited to these examples by any means.

### EXAMPLES

### <Method of Experiment>

### 1. Method for culturing induced pluripotent stem cells (iPSCs)

Cryopreserved human iPSCs (QHJI01s04, obtained from Kyoto University) were immersed in warm water at 37°C for 2 minutes and allowed to thaw while being shaken by hand. A cell preservation solution was suspended in nine times the amount of StemFit AK03 medium (Ajinomoto Healthy Supply Co., Inc.), and the resulting mixture was centrifuged at 150 to 200 × g for 5 minutes. After removing the cell supernatant, the cells were suspended in AK03 medium supplemented with 10 µM Y-27632 and 0.22 to 0.25 g/cm² iMatrix-511MG (Nippi, Incorporated) and seeded to a 90 mm dish. The medium was replaced with AK03 medium on day 1 of culture, after which medium replacement was performed on days 3, 5, and 6 of culture. For subculturing, the human iPSCs on day 7 of culture were washed with PBS, then 2 ml of Accutase (Innovative Cell Technologies) was added thereto, and the cells were treated with the Accutase at 37°C for 5 to 10 minutes to detach the cells. After adding 2 ml of AK03 medium, the cells were transferred to a 15 ml tube and then centrifuged at 150 to 200 × g for 5 minutes. After removing the cell supernatant, the cells were suspended in AK03 medium supplemented with 10 µM Y-27632 and 0.22 to 0.25 g/cm² iMatrix-511MG and seeded to a 90 mm dish. The cells were cultured until day 7 of culture in the same manner as that described above for day 1 of culture onwards.

### 2. Method for inducing differentiation from iPS cells to liver progenitor cells

Human iPSCs that had been cultured for 7 days were washed with PBS, then 2 ml of Accutase was added thereto, and the cells were treated with the Accutase at 37°C for 5 to 10 minutes to detach the cells. After cell collection and centrifugation, the cell supernatant was removed, and the cells in a required amount were suspended in 600 to 1,000 µl of AK03N medium and added directly to a 90 mm dish that had been coated from the previous day with 9 ml of RPMI medium supplemented with 100 units/ml penicillin, 100 µg/ml streptomycin, 20% StemFit For Differentiation (Ajinomoto Healthy Supply Co., Inc.), 2 µM CHIR99021, 33 µg/ml Activin A, 10 µM Y-27632, and 0.22 to 0.25 µg/cm² iMatrix-511MG. On day 1 of culture, the medium was replaced with RPMI medium supplemented with 100 units/ml penicillin, 100 µg/ml streptomycin, 20% StemFit For Differentiation, 2 µM CHIR99021, 33 µg/ml Activin A, and 500 µM sodium butyrate. On day 3 of culture, the medium was replaced with RPMI medium supplemented with 100 units/ml penicillin, 100 µg/ml streptomycin, 20% StemFit For Differentiation, 33 µg/ml Activin A, and 500 µM sodium butyrate. On day 4 of culture, the medium was replaced with RPMI medium supplemented with 100 units/ml penicillin, 100 µg/ml streptomycin, 20% StemFit For Differentiation, and 33 µg/ml Activin A. On day 5 of culture, the medium was replaced with StemFit Basic03 (Ajinomoto Healthy Supply Co., Inc.) supplemented with 100 units/ml penicillin, 100 µg/ml streptomycin, 1 × NEAA (MEM Non-Essential Amino Acids Solution), 1% dimethyl sulfoxide, 1 mM L-Glutamine, and 100 µM 2-mercaptoethanol, after which medium replacement was performed every day until day 9 of culture. The cells on day 10 of culture were used as hepatic progenitor cells (HEs).

### 3. Method for inducing differentiation from iPS cells to vascular endothelial cells

Human iPSCs that had been cultured for 7 days were washed with PBS, then 2 ml of Accutase was added thereto, and the cells were treated with the Accutase at 37°C for 5 to 10 minutes to detach the cells. After cell collection and centrifugation, the cell supernatant was removed, and the cells were suspended in AK03 medium supplemented with 10 µM Y-27632 and 0.22 to 0.25 µg/cm² iMatrix-511MG and then seeded into a 90 mm dish. On day 1 of culture, the medium was replaced with DMEM/F12 medium supplemented with 100 units/ml penicillin, 100 µg/ml streptomycin, 20% StemFit For Differentiation, 1 × GlutaMAX (ThermoFisher Scientific), 8 µM CHIR99021, and 20 ng/ml BMP-4, after which medium replacement was performed on day 3 of culture. On day 4 of culture, the medium was replaced with StemPro-34 medium (ThermoFisher Scientific) supplemented with 100 units/ml penicillin, 100 µg/ml streptomycin, 200 ng/ml VEGF, and 2 µM forskolin, after which medium replacement was performed every day until day 9 of culture. The cells on day 10 of culture were washed with PBS, then 2 ml of 0.05% Trypsin-EDTA was added thereto, and the cells were treated with the 0.05% Trypsin-EDTA at 37°C for 5 to 10 minutes to detach the cells. After cell collection and centrifugation, the cell supernatant was removed, and the cells were suspended in StemPro-34 medium supplemented with 100 units/ml penicillin, 100 µg/ml streptomycin, 50 ng/ml VEGF, and 0.22 to 0.25 µg/cm² iMatrix-511MG and then seeded into a 90 mm dish. The medium was replaced with MiraCell EC Culture Medium (TAKARA BIO INC.) on day 11 of culture, after which medium replacement was performed every day until day 16 of culture. The cells on day 17 of culture were used as vascular endothelial cells (ECs).

### 4. Method for inducing differentiation from iPS cells to mesenchymal stem cells

Human iPSCs that had been cultured for 7 days were washed with PBS, then 2 ml of Accutase was added thereto, and the cells were treated with the Accutase at 37°C for 5 to 10 minutes to detach the cells. After cell collection and centrifugation, the cell supernatant was removed, and the cells were suspended in AK03 medium supplemented with 10 µM Y-27632 and 0.22 to 0.25 µg/cm² iMatrix-511MG and then seeded into a 90 mm dish. On day 1 of culture, the medium was replaced with DMEM/F12 medium supplemented with 100 units/ml penicillin, 100 µg/ml streptomycin, 20% StemFit For Differentiation, 1 × GlutaMAX-I (ThermoFisher Scientific), 8 µM CHIR99021, and 50 ng/ml BMP-4, after which medium replacement was performed on day 3 of culture. On day 4 of culture, the medium was replaced with DMEM/F12 medium supplemented with 100 units/ml penicillin, 100 µg/ml streptomycin, 20% StemFit For Differentiation, 1 × GlutaMAX, 10 ng/ml PDGF-BB, and 0.66 ng/ml Activin A, after which medium replacement was performed every day until day 5 of culture. The cells on day 6 of culture were washed with PBS, then 2 ml of 0.05% Trypsin-EDTA was added thereto, and the cells were treated with the 0.05% Trypsin-EDTA at 37°C for 5 to 10 minutes to detach the cells. After cell collection and centrifugation, the cell supernatant was removed, and the cells were suspended in DMEM/F12 medium supplemented with 100 units/ml penicillin, 100 µg/ml streptomycin, 20% Stemfit For Differentiation, 1 × GlutaMAX, 10 ng/mL PDGF-BB, 0.66 ng/ml Activin A, and 10µM Y-27632 and then seeded into a 90 mm dish previously coated with 0.1% gelatin. On day 7 of culture, the medium was replaced with DMEM/F12 medium supplemented with 100 units/ml penicillin, 100 µg/ml streptomycin, 20% StemFit For Differentiation, 1 × GlutaMAX, 10 ng/ml bFGF, and 12 ng/ml BMP-4, after which medium replacement was performed every day until day 9 of culture. The cells on day 10 of culture were used as mesenchymal stem cells (MSCs). The MSCs were washed with PBS, then 2 ml of 0.05% Trypsin-EDTA was added thereto, and the cells were treated with the 0.05% Trypsin-EDTA at 37°C for 5 to 10 minutes to detach the cells. After cell collection and centrifugation, the cell supernatant was removed, and the cells were resuspended in STEM-CELLBANKER (Nippon Zenyaku Kogyo Co., LTD.) and cryopreserved at -80°C.

### 5. Fluorescent staining of cells

In order to observe cell distributions to be described below, the ECs were fluorescently stained using a PKH67 Green Fluorescent Cell Linker Mini Kit (Sigma-Aldrich) and the MSCs were fluorescently stained using Vybrant DiD cell-labeling solution (Thermo Fisher Scientific). The ECs on day 10 of culture were washed with PBS, then 2 ml of 0.05% Trypsin-EDTA was added thereto, and the cells were treated with the 0.05% Trypsin-EDTA at 37°C for 5 to 10 minutes to detach the cells. After cell collection and centrifugation, the cell supernatant was removed, and the cells were suspended in DMEM medium supplemented with 10% FBS. The collected ECs were fluorescently stained using the standard protocol for the PKH67 Kit (staining reaction time: 5 minutes), and the labeled ECs were resuspended in DMEM medium supplemented with 10% FBS. Thereafter, a cell count was taken.

The cryopreserved MSCs were immersed in warm water at 37°C for 3 minutes and allowed to thaw while being shaken by hand. A cell preservation solution was suspended in DMEM medium supplemented with 10% FBS and centrifuged at 150 to 200 × g for 5 minutes. The collected MSCs were fluorescently stained using the standard protocol for the Vybrant DiD cell-labeling solution (staining reaction time: 20 minutes), and the labeled MSCs were resuspended in DMEM medium supplemented with 10% FBS. Thereafter, a cell count was taken. The stained ECs and MSCs were used in production of cell-filled hollow microfibers and production of spheroids using an Elplasia plate, both of which will be described below.

### 6. Collection of HEs

The HEs on day 10 of culture were washed with PBS, then 2 ml of 0.05% Trypsin-EDTA was added thereto, and the cells were treated with the 0.05% Trypsin-EDTA at 37°C for 3 to 10 minutes to detach the cells. After cell collection and centrifugation, the cell supernatant was removed, and the cells were resuspended in DMEM medium supplemented with 10% FBS. Thereafter, a cell count was taken. The collected HEs were used in production of cell-filled fibers and production of spheroids using an Elplasia plate, both of which will be described below.

### 7. Production of cell-filled hollow microfibers

KBM VEC-1 Basal Medium (KOHJIN BIO) supplemented with KBM VEC-1 Supplement (KOHJIN BIO) and DMEM medium were mixed together at a ratio of 1:1, and the resulting medium was supplemented with 2.5% FBS, 50 nM dexamethasone, 10 ng/ml oncostatin M, and 10 µM Y-27632 to prepare a medium (LB medium). The HEs, the PKH67-labeled ECs, and the DiD-labeled MSCs were suspended in the LB medium containing or not containing 0.3% (w/v) methylcellulose at a ratio of 10:7:1 (HEs:PKH67-labeled ECs:DiD-labeled MSCs), and hollow microfibers with an inner diameter of 200 µm, in which 5.4 × 10⁵ cells in total were contained at a cell density of 1 × 10⁷ cells/ml, 5 × 10⁷ cells/ml, or 10 × 10⁷ cells/ml were produced. The hollow microfibers were produced according to the method for producing microfibers described in Japanese Patent No. 5633077.

### 8. Culture of hollow microfibers filled with cells

The hollow microfibers filled with 5.4 × 10⁵ cells at the respective cell densities were added to separate single wells of a 24-well plate containing 0.5 ml of LB medium, and the cells were cultured. On day 1 of culture, 0.5 ml of LB medium was added, and from day 2 to day 6 of culture, 0.5 ml of the medium was replaced every day.

### 9. Preparation of spheroids using Elplasia plate (control)

The HEs, the PKH67-labeled ECs, and the DiD-labeled MSCs were suspended in LB medium at a ratio of 10:7:1 (HEs:PKH67-labeled ECs:DiD-labeled MSCs) and then seeded to a 24-well Elplasia plate (Corning Incorporated) at a total cell count of 5.4 × 10⁵ cells/0.5 mL/well. The cells were cultured for 7 days. On day 1 of culture, 0.5 ml of LB medium was added, and from day 2 to day 6 of culture, 0.5 ml of the medium was replaced every day.

### 10. Observation of cell morphology and distribution

The cells were observed under a fluorescence microscope (KEYENCE CORPORATION) to examine the cell morphology and distribution of the ECs and the MSCs.

### <Results>

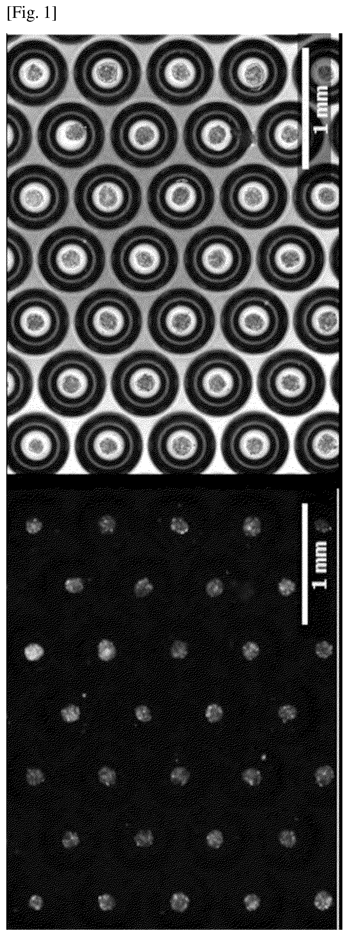

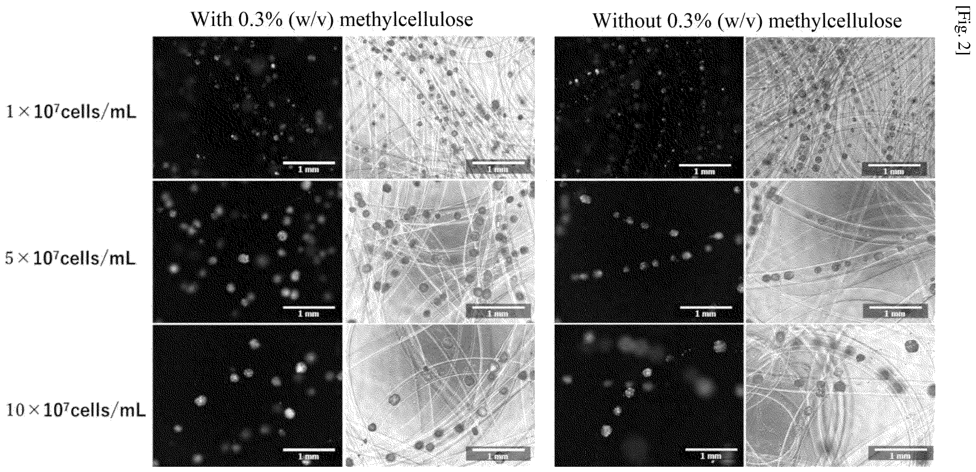

For the spheroids produced using the Elplasia plate (control) and the cells filled in the hollow microfibers, the cell morphology and the cell distribution were examined using a fluorescence microscopy on day 7 of culture. The spheroids produced using the Elplasia plate were all spherical with a size of 500 µm or less, and the fluorescence observation revealed that the ESCs (red) and the MSCs (green) were distributed evenly (FIG. 1). For the cells filled in the hollow microfibers, it was confirmed that spherical spheroids with a diameter of 500 µm or less were formed in the microfibers at all the cell concentrations. Further, the fluorescence observation confirmed that the ECs (red) and the MSCs (green) were distributed evenly in each spheroid (FIG. 2).

### 11. Examination of albumin secretion ability

On day 7 of culture, the medium for the spheroids produced using the Elplasia plate (control) and the medium for the cells filled in the hollow microfibers were removed and replaced with fresh media. 24 hours later, the culture supernatants were collected, and the albumin concentrations in the culture supernatants were measured using a human albumin ELISA Kit (Bethyl Laboratories).

### <Results>

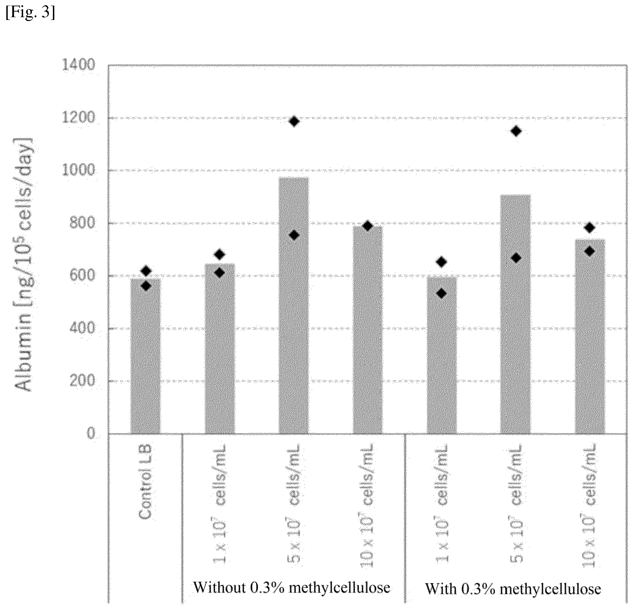

The amounts of albumin secretion per unit cells are shown (FIG. 3). Albumin secretion was observed under all the conditions. The amount of albumin secretion per unit cells was roughly equivalent to that of Control LB at the intermediate density (5 × 10⁷ cells/ml). Since albumin is secreted only from mature hepatocytes, this suggests that hepatic progenitor cells present in the cell aggregates had matured and albumin was secreted from the cell aggregates.

### INDUSTRIAL APPLICABILITY

According to the present invention, cell aggregates containing a plurality of types of cells can be produced efficiently and in large quantities in hollow microfibers. Besides, the cell aggregates produced by the present invention are capable of functioning as organoids and thus are very useful as an agent for transplantation therapy for use in vivo.

The present application claims priority based on Japanese Patent Application No. 2022-126643 filed in Japan (filing date: August 8, 2022), the disclosure of which is incorporated herein in its entirety by reference.

## Claims

1. A method for producing a cell aggregate, comprising the step of:
forming a cell aggregate by filling a mixture of mesenchymal stem cells, vascular endothelial cells, and organ cells into a microfiber and subjecting the cells to floating culture within the microfiber.

2. The method according to claim 1, wherein the cell aggregate is an organoid.

3. The method according to claim 1 or 2, wherein the organ cells are endodermal cells.

4. The method according to any one of claims 1 to 3, wherein the endodermal cells are hepatocytes.

5. The method according to any one of claims 1 to 4, wherein the microfiber has an inner diameter from 20 to 300 µm.

6. The method according to any one of claims 1 to 5, wherein a cell density in the microfiber filled with the cells is from 5 × 10⁶ cells/mL to 4 × 10⁸ cells/mL.

7. The method according to any one of claims 1 to 6, wherein a proportion of the mesenchymal stem cells in a cell population filling the microfiber is not less than 1% and not more than 20%.

8. The method according to any one of claims 1 to 7, wherein a proportion of the vascular endothelial cells in a cell population filling the microfiber is not less than 10% and not more than 60%.

9. The method according to any one of claims 1 to 8, wherein at least one type of cells selected from the mesenchymal stem cells, the vascular endothelial cells, and the organ cells are derived from induced pluripotent stem cells.

10. A cell aggregate produced by the method according to any one of claims 1 to 9.

11. An agent for transplantation therapy, comprising:
the cell aggregate according to claim 10.
